# EUROPÄISCHE PATENTSCHRIFT

(11) **EP 3 000 436 B1**
(45) Veröffentlichungstag und Bekanntmachung des Hinweises auf die Patenterteilung: **05.04.2017**
(21) Anmeldenummer: 14186172.4
(22) Anmeldetag: 24.09.2014
(51) Int. Cl.: A61F 2/24, A61F 2/95

(54) **System zum Ersatz einer an einer Entzündung oder einer Infektion erkrankten Klappe des Herzens**
System for replacing an inflamed or infected valve of the heart
Système de remplacement d'une valve cardiaque atteinte d'une inflammation ou d'une infection

(43) Veröffentlichungstag der Anmeldung: 30.03.2016
(73) Patentinhaber: Lauten, Alexander, 07743 Jena (DE); Figulla, Hans Reiner, 07749 Jena (DE)
(72) Erfinder: Lauten, Alexander, 07743 Jena (DE); Figulla, Hans Reiner, 07749 Jena (DE)
(74) Vertreter: Trinks, Ole

(56) Entgegenhaltungen:
- WO-A2-2008/070797
- US-A1- 2005 228 496
- US-A1- 2013 331 929
- US-A1- 2014 114 407

## Beschreibung

Die Erfindung betrifft ein System zum Ersatz einer an einer Entzündung und/oder einer Infektion erkrankten Klappe des Herzens.

Eine Entzündung und/oder eine Infektion des Herzens, eine sogenannten Endokarditis, wird in den meisten Fällen durch Bakterien wie Streptokokken, Staphylokokken oder Enterokokken ausgelöst. Abhängig von der Art des Auslösers, tritt eine mortale Folge der Endokarditis in bis zu 25% der Fälle ein. Gleichfalls weisen einmalig betroffene Patienten ein vielfach erhöhtes Risiko für eine erneute Endokarditis auf. Bakterien treten beispielsweise durch Verletzungen in der Mundhöhle, (postoperative) Wundflächen oder im Zuge von fieberhaften Erkrankungen in die Blutbahn ein. Folglich können Verwirbelungen des Blutstroms, u.a. infolge eines angeborenen Herzfehlers oder von Kalzifikationen, zu Verletzungen des Endokards oder der Herzklappen führen, die Ausgangspunkt einer Endokarditis sein können.

Die Prophylaxe sowie die Behandlung einer Endokarditis sieht die Einnahme von Antibiotika durch den Patienten über einen hinreichend langen Zeitraum vor. Aufgrund der geringfügigen Vaskularisation des Herzklappengewebes, kann ein bakterieller Befall der Herzklappen von der körpereigene Immunabwehr lediglich in begrenzten Umfang bekämpft werden. Es können pathologische Veränderungen an den Herzklappen beispielsweise in Form von Vernarbungen und weiteren Degenerationen entstehen, die fortfolgend eine Herzklappeninsuffizienz begründen können.

Eine Herzklappeninsuffizienz beschreibt die unzureichende Schlussfähigkeit der betroffenen Segelklappen bzw. Taschenklappen. Während der zyklisch auftretenden Diastole des Herzens entsteht somit ein unkontrollierter Rückfluss des Blutes, der eine unzureichende Versorgung des Körpers mit sauerstoffreichem Blut bedingt. Der Körper versucht anhand physiologischer Regelmechanismen der mangelnden Blutversorgung entgegenzuwirken, woraus mittel- und langfristig weitere Folgeerkrankungen auf Basis einer Hypertrophie des Herzens durch ein verkleinertes Herzzeitvolumen und hohe Blutdruckamplituden folgen. In Abhängigkeit von der jeweils betroffenen Herzklappe, können sich weitere Symptome beispielsweise in Form eines Lungenödems oder Wassereinlagerungen in den Gliedmaßen darstellen.

Eine Möglichkeit zur Behandlung einer Endokarditis ist nach dem Stand der Technik die chirurgische Entfernung der entzündeten Herzklappe und die Implantation einer künstlichen Herzklappe bzw. Ersatzherzklappe. In der Regel werden hierfür arbeits- und kostenintensive Operationen durchgeführt, die mit einer hohen Patientenbelastung und einem beträchtlichen Risiko verbunden sind. Im Detail wird der Brustkorb des Patienten eröffnet, das Herz mittels kardiopleger Lösung zum Stillstand gebracht, die körpereigene Herzklappe entfernt und an dessen Stelle eine künstliche Herzklappe an das körpereigene Gewebe angenäht. Neuere Methoden, wie in WO2006/076890 A1 dargestellt, sehen eine Transkatheter-Implantation künstlicher Herzklappen unter Einsatz eines Stents als Stützstruktur vor. WO 2008/070797 zeigt eine Herzklappe mit einer antimikrobiellen Beschichtung.

Der vorliegenden Erfindung liegt die Problemstellung zugrunde, dass sich bekannte Systeme nicht zur Behandlung eines an einer Endokarditis erkrankten Herzens eignen, da sie nicht zur Abgabe antimikrobieller, antibiotischer, bakterizider und/oder vergleichbarer Wirkstoffe an das umliegende Gewebe befähigt sind und daher keine Behandlung des Entzündungsprozesses erlauben. Neben einem verringerten Behandlungsrisiko für den Patienten und einer optimalen Passgenauigkeit einer langlebigen Ersatzherzklappe zur Behandlung einer Herzklappeninsuffizienz ergibt sich in diesem Zusammenhang das Problem, die fortschreitende Schädigung des Herzgewebes, durch den Entzündungsprozess zu verhindern. Im Weiteren zeigt sich beim Herzklappenersatz nach dem Transkatheter-Verfahren in ca. 40% der Fälle eine paravalvuläre Undichtigkeit am implantierten System.

Hinsichtlich dieser Problemstellung hat die vorliegende Erfindung die Aufgabe zu erfüllen, eine Möglichkeit zur gezielten, interventionellen Therapie für ein an einer Endokarditis erkranktes Herz bereitzustellen. Hierbei müssen die morbiden Folgen einer Endokarditis, insbesondere eine Herzklappeninsuffizienz, mit der vorliegenden Erfindung behandelbar sein. Gleichfalls muss die Entzündung und/oder Infektion direkt vor Ort behandelt und die Ausbreitung der Infektion kontrolliert werden können, ohne dass eine zusätzliche Belastung und Gefährdung des Patienten entsteht.

Diese Aufgabe wird dadurch gelöst, dass das erfindungsgemäße System ausgelegt ist, antimikrobielle, antibiotische, bakterizide, antiinflammatorische und/oder vergleichbare Wirkstoffe am Ort der Erkrankung an das umliegende Gewebe und in die Blutbahn abzugeben.

Dazu weist das erfindungsgemäße System ein Stent-Wirkstoff-System mit mindestens einem Stent auf, an dem eine Ersatzherzklappe befestigt ist. Insbesondere weist das beanspruchte System biokompatible Materialien auf, um eine gute Integrierbarkeit des Systems in das biologische Umfeld nach der Implantation zu gewährleisten. Der mindestens eine Stent stellt die Träger- und Stützstruktur der Ersatzherzklappe dar und dient gleichzeitig der Positionierung und Fixierung des erfindungsgemäßen Systems am Implantationsort. Auch ist der Einsatz einer Stent-in-Stent-Lösung als Stent-System vorstellbar. Dabei kann ein Stent-System aus mehr als einem Stent, insbesondere aus 2 oder 3 Stents, zur Verwendung im erfindungsgemäßen System vorgesehen werden.

Das Stent-Wirkstoff-System muss die insuffiziente, native Herzklappe radial verdrängen können, um die Ersatzherzklappe an dessen Stelle aufzuspannen und eine fehlerfreie Klappenfunktion während der Systole und Diastole des Herzens sicherzustellen. Auch muss der mindestens eine Stent dazu geeignet sein, während des periodischen Herzschlags der Ersatzherzklappe einen sicheren Halt zu bieten, sodass das erfindungsgemäße System nicht aufgrund wechselnder Druckverhältnissen im Herzen vom biologischen Gewebe, insbesondere von der Gefäßwand, abgelöst und vom Implantationsort ausgeschwemmt werden kann. Hierzu weist das erfindungsgemäße System mindestens einen Stent auf, der per Ballon-expansion mit Hilfe eines Ballonkatheters expandiert und am Implantationsort positioniert werden kann. Dabei wird der komprimierte und im Katheter verborgene Stent durch einen mit Flüssigkeit oder Gas zu füllenden Katheterballon expandiert. Alternativ kann der mindestens eine Stent des erfindungsgemäßen Systems ein selbstexpandierender Stent sein. Insbesondere besteht der Stent hierzu aus einer Formgedächtnislegierung, vorzugsweise aus Nitinol. Neben dem Formgedächtniseffekt bei einer spezifischen Sprungtemperatur, nahe der Körpertemperatur, weist Nitinol gleichfalls Superelastizität, Biokompatibilität und Korrosionsbeständigkeit auf. So wird Nitinol bereits vielfach in der Medizintechnik angewendet. Insbesondere die Superelastizität ist vorteilhaft hinsichtlich der komprimierten Zuführungsform eines Stents im Transkatheter-Verfahren und der Expansion am Implantationsort. Neben den zwei separat ausgeführten Expansionsverfahren ist gleichfalls auch eine Kombination beider Verfahren möglich. Insbesondere die radiale Vorspannkraft des Stents kann nach der Selbstexpansion durch eine Ballonexpansion noch zusätzlich gesteigert werden, wobei wiederrum eine höhere Stabilität des erfindungsgemäßen Systems im implantierten Zustand erreicht wird.

Die an dem mindestens einen Stent befestigte Ersatzherzklappe kann eine Perikardklappe sein, eine Schweineherzklappe, eine künstliche Herzklappe, vorzugsweise bestehend aus biokompatiblen Materialien, oder ein vergleichbares Implantat oder Transplantat, welches zum Ersatz einer insuffizienten Herzklappe geeignet ist. Somit bietet das System den Vorteil, in Abhängigkeit von den patientenspezifischen Bedingungen die optimale Ausführung einer Ersatzherzklappe aufweisen zu können. Des Weiteren weist die Ersatzherzklappe mindestens zwei Leaflets auf. Hinsichtlich des Ersatzes einer dreiteiligen Herzklappe ist auch der Einsatz mit mehr als zwei, insbesondere mit drei Leaflets, vorstellbar. Somit ist die Verwendung des erfindungsgemäßen Systems nicht nur auf den Ersatz einer insuffizienten, nativen Aortenklappe begrenzt, insbesondere nicht durch die Anzahl der Leaflets.

Die Leaflets der Ersatzherzklappe weisen in ihrer vorgesehenen Verwendung insbesondere zwei Positionen auf, die sie während der Systole und der Diastole des Herzens einnehmen. Mit dem Ziel eine native Herzklappe als biologisches Vorbild zu imitieren, ist eine äquivalente Übertragung der Funktionalität der Leaflets dem biologischen Vorbild entsprechend auch für den Ersatz der weiteren nativen Herzklappen vorstellbar. In einer ersten Position der Leaflets, während der Diastole des Herzens, wird die strömungsmäßige Verbindung zwischen linkem Ventrikel und Aorta vollständig abgeschlossen, sodass ein Blutrückfluss verhindert wird. Hierbei weisen die Kommissuren der Leaflets, die gefäßinnenseitig liegenden Kanten, Kontakt zueinander auf. Während der Systole des Herzens nehmen die Leaflets eine zweite, geöffnete Position ein, sodass das Blut vom Ventrikel aus in die Aorta gefördert werden kann. Die Kommissuren der Leaflets weisen in dieser zweiten Position keinen Kontakt zueinander auf.

Der mindestens eine Stent weist an der Innenseite und/oder Außenseite eine Beschichtung auf, die aus einer antimikrobiellen Substanz oder einem antimikrobiell wirkenden Trägermaterial besteht. Somit kann der mindestens eine Stent antimikrobielle Wirkstoffe freisetzen und eine verbesserte Integrierbarkeit des implantierten System im Kontakt mit der umliegenden Gefäßwand erzielen. Insbesondere bei Einsatz eines antimikrobiell wirkenden Trägermaterials ist eine Kombination mit weiteren Komponenten wie Antikoagulantien, weiteren antimikrobiellen Wirkstoffen wie Bakteriziden, etc. möglich, die an und/oder auf dem Trägermaterial platziert werden. Um derartige Beschichtungen erzeugen zu können, sind u.a. die Aufbringung einer Folie auf der Stent-Oberfläche sowie weitere physikalische und/oder chemische Ablagerungsverfahren zur Aufbringung einer Oberflächenbeschichtung für das erfindungsgemäße System anwendbar. Es ist somit möglich, an der Stent-Oberfläche eine komplexe Freisetzungsdynamik vorzugsweise antimikrobieller Wirkstoffe zu erzielen.

Eine Beschichtung der Stent-Oberfläche kann in einer weiteren Ausführungsform kontrolliert aktivierbar sein. Dabei tritt ein vorzugsweise antimikrobieller Effekt erst ein, wenn eine Aktivierung der Oberflächenbeschichtung erfolgt ist. Vorzugsweise kann eine derartige, kontrollierte Aktivierung der Oberflächenbeschichtung durch die Applikation von Ultraschall von außerhalb des Körpers des Patienten erfolgen, wobei mindestens eine, an spezielle Trägermedien gebundene, toxische Substanze, wie beispielsweise Kohlenstoffdioxid, an der Stent-Oberfläche freigesetzt wird.

Zusätzlich hierzu weist das erfindungsgemäße System einen ersten Schürzenbereich innerhalb und einen zweiten Schürzenbereich außerhalb des vorgesehenen Stents auf, wobei eine eingefüllte Substanz im ventrikelseitigen Retentionsbereich des Stents freigesetzt wird. Insbesondere wird unter dem ventrikelseitigen Retentionsbereich im Falle eines Aortenklappenersatzes der Retentionsbereich eines erfindungsgemäßen Stents bzw. Stent-Systems verstanden, der dem linken Ventrikel des Herzens zugewandt und dem aortaseitigen Retentionsbereich gegenübergestellt ist. Somit ergibt sich ein Blutfluss durch das erfindungsgemäße System, wobei eine Substanz am ventrikelseitigen Bluteingang des Systems freigesetzt wird, während der Blutstrom auf der Blutausgangsseite des erfindungsgemäßen Systems, am aortaseitigen Retentionsbereich, in die Aorta übergeht. Entsprechend der erfindungsgemäßen Verwendung des beanspruchten Systems, ist auch bei Ersatz anderer Herzklappen die Freisetzung der Substanz stets auf der Bluteingangsseite des erfindungsgemäßen Systems anzunehmen.

Die mindestens eine Kammer in oder an dem Schürzenbereich kann sowohl am inneren Radius (Innenseite) als auch am äußeren Radius (Außenseite) des mindestens einen Stents vorliegen. In Abhängigkeit vom benötigten Volumen zur Einfüllung einer hinreichenden Menge der mindestens einen Substanz ist es vorstellbar, dass sich neben dem Volumen an der Außenseite auch an der Innenseite des mindestens einen Stents ein Volumen zur Aufnahme einer Substanz befindet. Eine Vergrößerung des Substanzvolumens kann zur Maximierung der Behandlungsdauer mit einer Substanz oder zur Erhöhung der Behandlungsintensität beitragen. Insbesondere ist auch vorstellbar, dass eine räumliche Trennung, z.B. durch eine nicht permeable, eine permeable oder eine selektiv permeable Membran vorliegt, sodass prinzipiell zwei getrennte Kammern am Außen- und Innendurchmesser des mindestens einen Stents bestehen. Entsprechend könnte eine nicht freizusetzende Substanz in der Kammer am Außendurchmesser des mindestens einen Stents verbleiben, während eine weitere Substanz am Innendurchmesser in den Blutstrom freigesetzt wird, insbesondere zur interventionellen Behandlung einer Endokarditis. Gleichzeitig besteht auch die Möglichkeit, eine Diffusion über eine permeable oder selektiv permeable Membran zu implementieren und die Freisetzung der Substanz ausschließlich über die Kammer am Innendurchmesser des Stents erfolgen zu lassen. Somit stellt eine Kammer, die sich am inneren und äußeren Radius des Stents erstreckt, ein wesentlich größeres Volumen zur Aufnahme und Freisetzung von Substanzen zur Verfügung.

Des Weiteren kann ein Schürzenbereich an der Außenseite des Stents mit der mindestens einen, mit einer Substanz gefüllten Kammer gleichzeitig als ein Dichtungsmittel dienen. In Kontakt mit einer Gefäßwand kann das mit einer Substanz befüllte System somit die paravalvuläre Dichtigkeit des Systems unabhängig von der patientenspezifischen Anatomie gewährleisten. Die Gefäßwand stellt im Kontext der vorliegenden Erfindung neben dem Gewebe der Aorta auch das biologische Gewebe einer nativen Herzklappe und des Herzens dar. Insbesondere der Schürzenbereich mit mindestens einer Kammer besitzt im mit einer Substanz befüllten Zustand ein Volumen, welches einen unkontrollierten Blutstrom am seitlichen Rand des erfindungsgemäßen Stents während der Systole und/oder der Diastole verhindern kann. Unter einem unkontrollierten Blutstrom ist hierbei insbesondere der Rückfluss von Blut entgegen der anatomisch korrekten Flussrichtung zu verstehen. Aufgrund einer besseren Abdichtung des implantierten Systems erhöht sich gleichzeitig dessen Effizienz in Hinblick auf die Imitation einer nativen Herzklappe. Gleichzeitig wird ein Ablösen des erfindungsgemäßen Systems und ein Ausschwemmen vom Implantationsort, beispielsweise aufgrund wechselnder Druckverhältnisse im Herzen, verhindert.

Weiterhin sieht die vorliegende Erfindung vor, dass die mindestens eine Kammer vor und/oder während und/oder nach der Implantation des mindestens einen Stents mit einer Substanz befüllbar ist. Somit ist es möglich, dass die mindestens eine Kammer bereits im komprimierten Zustand vor der Implantation eine Substanz beinhaltet. Außerdem ist vorgesehen, dass die Kammer sowohl während als auch nach der Implantation des mindestens einen Stents mit einer Substanz befüllt werden kann. Dabei können vor und/oder während und/oder nach der Implantation auch Substanzen unterschiedlicher Art und/oder Zusammensetzung in die mindestens eine Kammer eingefüllt werden. Sofern mehrere Kammern existieren, ist auch die Befüllung der Kammern mit verschiedenen Substanzen bzw. Substanzen mit unterschiedlicher Komponentenzusammensetzung durch die vorliegende Erfindung nicht ausgeschlossen. Folglich ist die Freisetzung verschiedener Wirkstoffe, insbesondere in sequentieller Weise, möglich und es kann eine komplexe Wirkstofffreisetzung zur Behandlung einer Entzündung und/oder Infektion am Herzen appliziert werden. Dementsprechend vielseitig und individuell kann eine patientenspezifische Behandlung mit Wirkstoffen in Hinblick auf Art und/oder Intensität und/oder Dauer der Wirkstofffreisetzung erfolgen.

Des Weiteren weist eine Ausführungsform der vorliegenden Erfindung eine Schürze auf, die als permeable, insbesondere als selektiv permeable Membran, ausgestaltet ist. Sofern eine Kammer mit einer Substanz, bestehend aus verschiedenen Fluiden und/oder Komponenten und/oder Wirkstoffen, befüllt wird ist es folglich möglich, dass lediglich ein Teil der eingefüllten Substanz intra- und/oder postoperativ freigesetzt wird. Ebenfalls ist der Einsatz von mehreren Fluiden unterschiedlicher Viskosität oder der Einsatz von Wirkstoffen mit verschiedenen Diffusions- bzw. Freisetzungscharakteristika denkbar. Die therapeutischen Maßnahmen zur Behandlung eines an einer Endokarditis erkrankten Herzens können auf diesem Wege individuell auf die spezifischen Bedingungen eines Patienten abgestimmt werden.

Auch kann die Kammer im Schürzenbereich die Freisetzung einer Substanz beeinflussen, indem sie, insbesondere teilweise, ein biologisch degradierbares und/oder resorbierendes Material, beispielsweise in Form von Oberflächenbeschichtungen und/oder Matrizen, aufweist. Entsprechend kann die Freisetzung mindestens einer Substanz insbesondere zu einem spezifischen Zeitpunkt bzw. nach einer spezifischen Degradationszeit eingesetzt werden oder eine Abfolge zur sequentiellen Freisetzung verschiedener Substanzen in das erfindungsgemäße System implementiert werden. Es ergibt sich hieraus die Möglichkeit einer komplexen Behandlungsstrategie, um eine Endokarditis mit Hilfe der vorliegenden Erfindung zu behandeln.

In Anbetracht der beschriebenen Freisetzungsmechanismen weist die vorliegende Erfindung verschiedene Möglichkeiten für eine individuelle Freisetzungsdynamik einer in mindestens eine Kammer eingefüllten Substanz auf. Insbesondere eine sequentielle, intraoperative Einfüllung verschiedener Komponenten einer Substanz sowie die spezifische Ausgestaltung und der Füllgrad der mindestens einen Kammer kann die Freisetzungsdynamik vor und/oder während und/oder nach einer Implantation beeinflussen. Neben der Freisetzung von Substanzen aus der mindestens einen Kammer kann gleichzeitig auch eine mindestens teilweise Beschichtung des gesamten erfindungsgemäßen Systems vorgesehen werden. Ebenso ermöglicht die Integration von resorbierenden und/oder biologisch degradablen Materialien als Oberflächenbeschichtung und/oder Matrizen, insbesondere an dem mindestens einen Stent und/oder innerhalb der mindestens einen Kammer, eine komplexe Freisetzungsdynamik von Wirkstoffen. Gleichfalls sollen derartige Komponenten die geometrischen Abmessungen des erfindungsgemäßen Systems für eine Implantation mittels Transkatheter-Verfahren im Wesentlichen nicht beeinflussen.

Die vorliegende Erfindung sieht außerdem eine Ausführungsform vor, wobei mindestens eine Kammer mindestens eine strömungsmäßige Verbindung zum ventrikelseitigen Retentionsbereich des mindestens einen Stents aufweist. Insbesondere dient die strömungsmäßige Verbindung dazu, die in die mindestens eine Kammer eingefüllt Substanz zum ventrikelseitigen Retentionsbereich des mindestens einen Stents zu leiten und dort freizusetzen. Dabei ist gleichfalls vorstellbar, dass die strömungsmäßige Verbindung einer Kammer eine Perforation in Längsrichtung aufweist, sodass die Substanz während des Strömungsverlaufes in Richtung des ventrikelseitigen Retentionsbereiches mindestens teilweise aus der strömungsmäßigen Verbindung austreten kann. Folglich kann die eingefüllte Substanz direkt vor Ort und vorzugsweise antimikrobiell auf die Entzündung und/oder Infektionen einwirken.

In einer weiteren erfindungsgemäßen Ausführungsform ist die eine, in mindestens eine Kammer einfüllbare Substanz eine antimikrobiell wirkende Substanz und/oder weist mindestens eine antimikrobiell wirkende Komponente auf. Die Substanz kann dabei Fluide und/oder Komponenten mit unterschiedlichen Viskositäten wie beispielsweise Antibiotika, Kochsalzlösung, Wachstumsfaktoren, Antikoagulantien etc. aufweisen. Insbesondere sollen antimikrobielle Wirkstoffe, wie z.B. Antibiotika, zur Behandlung einer Endokarditis hierunter verstanden werden. Generell beschreiben Antibiotika dabei Stoffe sowohl synthetischen als auch biogenen Ursprungs, insbesondere zur Bekämpfung bakterieller Infektionskrankheiten. Speziell im Falle der Befüllung der mindestens einen Kammer mit antimikrobiellen Wirkstoffen ergibt sich der Vorteil, dass ein an einer Entzündung und/oder Infektion erkranktes Herz nach dem Ersatz einer insuffizienten, nativen Herzklappe intra- und/oder postoperativ durch das erfindungsgemäße System medikamentös behandelt werden kann. In Abhängigkeit der eingefüllten Substanz und seiner Zusammensetzung kann somit kurz-, mittel- und langfristig die Wirkstoffabgabe vom erfindungsgemäßen System ausgehend erfolgen und eine Morbidität des Patienten behandelt werden. Des Weiteren ist es denkbar, dass eine Substanz aus einer Kombination verschiedener Komponenten bzw. Fluiden einen vollständigen Volumenverlust der mindestens einen Kammer verhindert indem eine Restvolumen in der mindestens einen Kammer verbleibt und somit die paravalvuläre Dichtigkeit aufrechterhalten werden kann.

In einer zusätzlichen Ausführungsform weist das erfindungsgemäße System außerdem ein Kathetereinführsystem auf. Ein derartiges Kathetereinführsystem dient zur Zuführung des erfindungsgemäßen Systems zum erkrankten Herzen sowie der Positionierung und Fixierung des mindestens einen Stents und der daran befestigten Ersatzherzklappe am Implantationsort mittels Ballonexpansion und/oder Selbstexpansion des mindestens einen Stents. Dabei wird zur Ballonexpansion ein Ballon hinter der Katheterspitze vorgesehen, der über im Katheterinneren liegende Lumen mit einem Fluid befüllt und durch einen hydrostatischen Druck expandiert werden kann. Die Lumen sind gleichfalls auch im Falle der Anwendung eines selbstexpandierenden Stents notwendig und werden während der Zuführung des erfindungsgemäßen Systems zum erkrankten Herzen mit kühler Flüssigkeit gespült. Die Auslösung der Selbstexpansion kann durch einen Spülstopp der kühlen Flüssigkeit erfolgen und/oder durch das Spülen der Lumen mit warmer Flüssigkeit. Weiterhin werden bei beiden Expansionsverfahren die verfügbaren Lumen eines Katheters vorzugsweise kontinuierlich gespült um einem Bluteintritt in das Kathetereinführsystem und/oder einem Gaseintritt in das vaskuläre System des Patienten vorzubeugen. In Abhängigkeit vom Zustand und den anatomischen Gegebenheiten des Patienten kann somit eine minimalinvasive Methode zur Implantation des erfindungsgemäßen Systems bereitgestellt werden, wobei die Implantation mit einer möglichst geringfügigen Patientenbelastung, verringerter Operationsdauer und reduzierten Behandlungskosten einhergeht.

Außerdem ist das Kathetereinführsystem sowohl zur transfemoralen als auch transapikalen Zuführung des mindestens einen Stents zum erkrankten Herzen geeignet. Speziell zur Durchführung der transfemoralen Methode muss das Kathetereinführsystem eine Flexibilität, insbesondere im distalen Bereich der Katheterspitze, als auch Bedienelemente zur Steuerung und Führung der Katheterspitze durch das vaskuläre System des Patienten aufweisen. Außerdem ist der Außendurchmesser des Kathetereinführsystems für die transfemorale Implantationsmethode auf die Dimensionen des vaskulären Systems begrenzt. Ebenso ist die Länge des mindestens einen Stents und der darauf befestigten Ersatzherzklappe sowie die Läng der Katheterspitze begrenzt, damit das erfindungsgemäße System im implantierten Zustand nicht mit dem Endokard bzw. dem Herzmuskel des schlagenden Herzens in Kontakt kommen kann. Zur Befüllung der mindestens einen Kammer des erfindungsgemäßen Systems mit mindestens einer Substanz sind außerdem Bedienelemente vorzusehen, mit denen die Befüllung der mindestens einen Kammer mit mindestens einer Substanz gezielt erfolgen kann. Mit Hilfe externer Bedienelemente sind somit diverse Vorteile einer minimalinvasiven Behandlung, insbesondere einer geringeren Patientenbelastung und geringerer Behandlungskosten, gegeben. Sofern das vaskuläre System oder der Zustand des Patienten, z.B. aufgrund zu geringer Gefäßdurchmesser, für das transfemorale Katheterverfahren ungeeignet ist, ist die Implantation des erfindungsgemäßen Kathetereinführsystem gleichfalls nach dem transapikalen Implantationsverfahren durchführbar.

Um die mindestens eine Kammer mit mindestens einer Substanz zu befüllen, weist eine Ausführungsform des Kathetereinführsystems mindestens einen Kanal auf, der zum Befüllen der mindestens einen Kammer dient. Der Kanal kann sowohl im Kathetereinführsystem als Lumen implementiert oder als separates Lumen außen am Kathetereinführsystem angelegt sein. Vorzugsweise liegt der Kanal jedoch als separates Lumen außerhalb des Kathetereinführsystems lösbar mit diesem verbunden vor. Insbesondere ist es in dieser bevorzugten Ausführungsform möglich, auch nach der Implantation des erfindungsgemäßen Systems die mindestens eine Kammer mit einer Substanz zu befüllen. Hierzu wird die lösbare Verbindung zwischen Kathetereinführsystem und dem mindestens einen Kanal getrennt und das Kathetereinführsystem aus dem vaskulären System des Patienten entfernt. Der mindestens eine Kanal verbleibt dabei innerhalb des vaskulären Systems des Patienten und stellt fortlaufend eine strömungsmäßige Verbindung zur Befüllung der mindestens einen Kammer mit einer Substanz, vorzugsweise von den externen Bedienelementen ausgehend, dar. Somit ist nach der Implantation des erfindungsgemäßen System und der Entfernung des Kathetereinführsystems auch weiterhin die Befüllung der mindestens einen Kammer mit einer Substanz möglich. Ein Patient kann entsprechend auch nach der Implantation mit vorzugsweise antimikrobiellen Wirkstoffen versorgt werden, um eine Entzündung und/oder eine Infektion des Herzens zu behandeln.

Des Weiteren stellt der Kanal eine strömungsmäßige Verbindung dar, die gleichfalls als Schläuche oder Schlauchverbindungen oder vergleichbare Verbindungen mit hinreichender Steifigkeit und Flexibilität zur Zuleitung von Fluiden ausgeführt sein kann. Insbesondere kann der Kanal in der Kammer mit einem Form-und/oder Kraftschluss befestigt sein, der vorzugsweise lösbar ausgestaltet ist. Demgemäß kann in einer Ausführungsform die mindestens eine strömungsmäßige Verbindung zu der mindestens einen Kammer vorzugsweise über ein Bedienelement des Kathetereinführsystems gezielt getrennt werden. Im Falle eines reinen Kraftschlusses, kann die strömungsmäßige Verbindung über eine definierte Zugkraft gelöst werden. Dabei kann u.a. mindestens eine Öffnung der Kammer bestehen bleiben durch die die mindestens eine Substanz aus der Kammer austreten und in das Blut übertreten kann. Alternativ kann die mindestens eine Öffnung nach der Entfernung des mindestens einen Kanals, vorzugsweise durch eine elastische Ausführungsform der Schürze, flüssigkeitsdicht abgeschlossen werden. Es ergibt sich der Vorteil, die Befüllung einer Kammer mit einer Substanz mittels dem Kathetereinführsystem gezielt und individuell ausführen zu können, wobei ein optimaler Therapieerfolg unter Anwendung des erfindungsgemäßen System gewährleistet ist.

Die vorliegende Erfindung beansprucht neben einem System außerdem ein Verfahren zum Ersatz einer an einer Entzündung und/oder einer Infektion erkrankten Klappe des Herzens. Hierbei wird das erfindungsgemäße System bereitgestellt und implantiert, um eine insuffiziente, native Herzklappe zu ersetzen und eine Endokarditis zu behandeln. Insbesondere sieht die Implantation des erfindungsgemäßen Systems die Zuführung zum erkrankten Herzen mit anschließender Expansion und Fixierung im Implantationsort vor. Vor und/oder während und/oder nach der Implantation des erfindungsgemäßen Systems kann die mindestens eine Kammer mit einer Substanz befüllt werden. Vorteilhafterweise ist somit die Einbringung und Freisetzung einer vorzugsweise antimikrobiell wirkenden Substanz aus der mindestens einen Kammer des erfindungsgemäßen Systems am Implantationsort kurz-, mittel- und langfristig möglich.

Im Folgenden werden exemplarische Ausführungsformen des erfindungsgemäßen Systems anhand der beigefügten Zeichnungen näher beschrieben. Weitere Ausführungsformen sollen durch die hier aufgeführten Beispiele nicht ausgenommen sein.
- Fig. 1:: Eine exemplarische Ausführungsform des Kathetereinführsystems mit expandiertem Stent mit einer darauf befestigten Ersatzherzklappe;
- Fig. 2:: Kathetereinführsystem mit Stent und darauf befestigter Ersatzherzklappe gemäß Fig. 1, im implantierten Zustand im Herzen;
- Fig. 3:: Kathetereinführsystem mit Stent und darauf befestigter Ersatzherzklappe gemäß Fig. 2, nach der Befüllung der Kammer im Schürzenbereich mit mindestens einer Substanz;
- Fig. 4:: Eine weitere exemplarische Ausführungsform des erfindungsgemäßen Systems in Form eines expandierten Stents mit einer darauf befestigten Ersatzherzklappe.

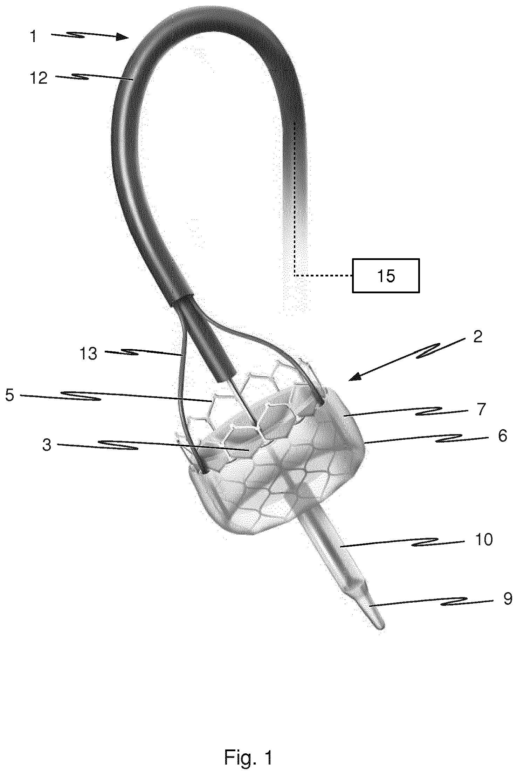

Nachfolgend wird eine exemplarische Ausführungsform des erfindungsgemäßen Systems 2 mit Bezug auf Fig. 1 näher erläutert. Dabei zeigt Fig. 1 das erfindungsgemäße System 2 im expandierten Zustand mit einem Stent 5, einer Ersatzherzklappe 3 mit mindestens zwei Leaflets 4 und einem Schürzenbereich 6 welcher im ventrikelseitigen Retentionsbereich des Stents 5 an dessen Innen- und Außenseite vorgesehen ist. In dem Schürzenbereich 6 ist eine Kammer 7 gezeigt, die mit mindestens einer Substanz befüllt werden kann. Hierzu verfügt das dargestellte Kathetereinführsystem 1 über einen Katheter 12 mit zwei Kanälen 13, die eine strömungsmäßige Verbindung zu der Kammer 7 herstellen.

Des Weiteren weist das Kathetereinführsystem in der gezeigten Ausführungsform einen Ballon 10 zur Ballonexpansion des Stents 5 und eine Katheterspitze 9 auf. Die Expansion des Stents 5 erfolgt indem dem Katheterballon 10 durch ein inneres Lumen des Katheters 12 ein Fluid zugeführt wird, sodass ein hinreichender hydrostatischer Druck zur Expansion des Stents 5 durch den Ballon 10 entsteht. Hierzu kann der Katheter aus mehreren Schichten bzw. Lumen aufgebaut sein, um die notwendige Funktionalität zu bieten. Im Anschluss an die Expansion des Stents 5 wird der Ballon wieder komprimiert, sodass der Katheter zum Abschluss der Operation aus dem vaskulären System des Patienten entfernt werden kann.

Weiterhin stellt der Katheter 12 einen flexiblen Katheter dar, der durch das vaskuläre System eines Patienten geführt werden kann, wobei die Spitze 9 mit den Bedienelementen 15 steuerbar ist, um transfemoral bis zum Herzen zugeführt werden zu können. Ebenso dienen die Bedienelemente insbesondere zur Kontrolle des erfindungsgemäßen Systems in Verbindung mit dem Kathetereinführsystem, z.B. zum Lösen des Katheters 12 vom System 2 nach dessen erfolgreicher Implantation am Implantationsort.

Zur Befüllung der Kammer 7 mit mindestens einer Substanz sind die Kanäle 13 mit der Kammer 7 verbunden. Dabei kann die Kammer 7 insbesondere mit einer Substanz, insbesondere mit einer Substanz aus mehreren Komponenten, insbesondere mit verschiedenen Substanzen sequentiell befüllt werden. Die Kammer 7 kann sich weiterhin an der Außenseite und/oder der Innenseite des Stents 5 ausbilden. Im Anschluss an die Befüllung der Kammer 7, können die Kanäle 13 per Zugkraft und/oder mit den Bedienelementen 15 gezielt vom System 2 gelöst werden. Die Freisetzung der eingefüllten Substanz aus der Kammer 7 in das Blut des Patienten kann über die verbliebenen, offenen Verbindungsstellen der Kanäle 13 oder anhand einer Diffusion der Substanz durch die Schürze (6) hindurch erfolgen.

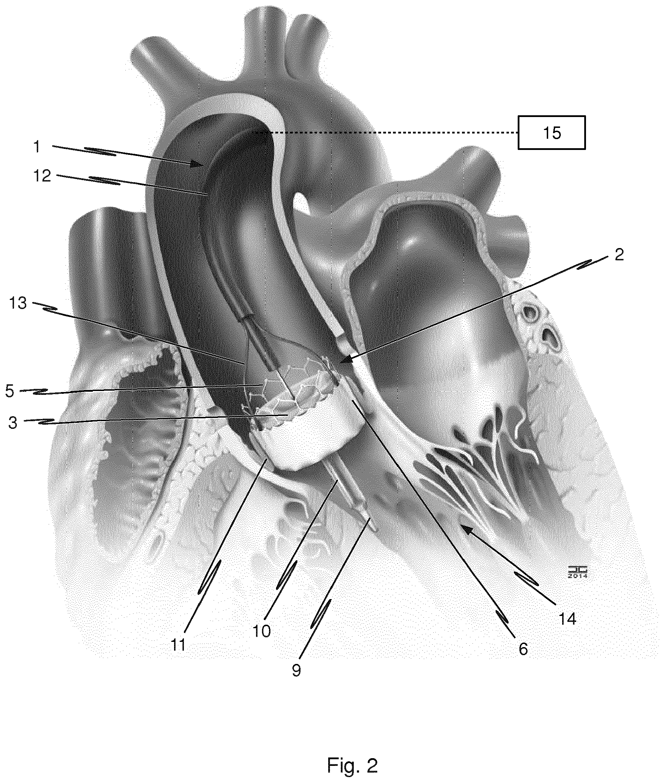

Fig. 2 veranschaulicht die Ausführungsform der vorliegenden Erfindung gemäß Fig. 1 bei der Implantation in einem erkrankten Herzen 14. Im Detail stellt Fig. 2 den expandierten Stent 5 mit der darauf befestigten Ersatzherzklappe 2 dar, wobei die insuffiziente, native Herzklappe 11 durch den expandierten Stent 5 radial verdrängt wird. Weiterhin verdeutlicht Fig. 2, dass das System 2 mit dem Stent 5, der Ersatzherzklappe 2 und dem Kathetereinführsystem 1 in seiner Längendimension begrenzt ist, um einen Kontakt mit dem Endokard des Herzens und dem Herzmuskel zu vermeiden.

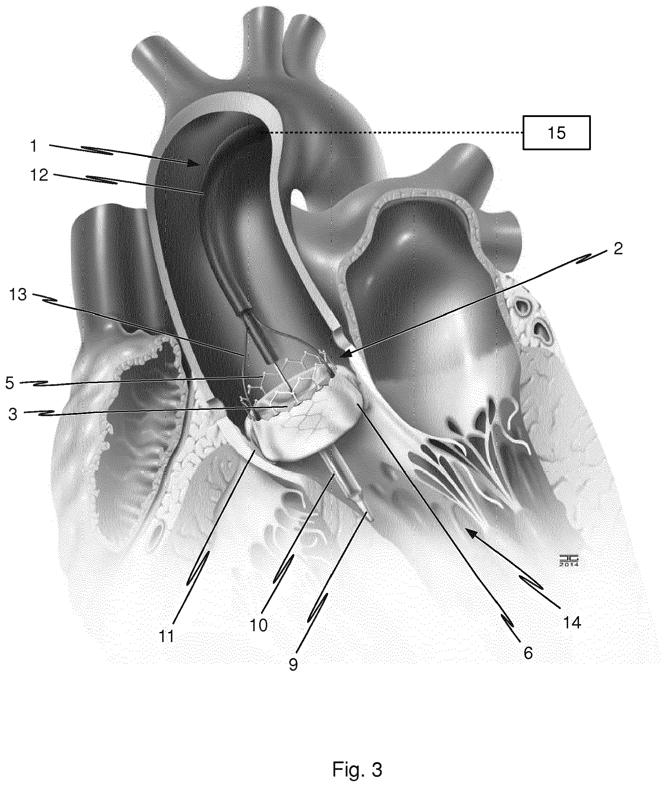

Fig. 3 illustriert die Implantation des Systems 2 gemäß Fig. 2, wobei die Kammer 7 mit mindestens einer Substanz befüllt wird. Es ist ersichtlich, dass die Schürze 6 durch die Volumenausdehnung der Kammer 7 bei der Befüllung mit mindestens einer Substanz, die paravalvuläre Dichtigkeit im Kontakt mit der Gewebe der insuffizienten, nativen Herzklappe 11 verbessert. Insbesondere ist es ebenfalls möglich, dass die Kammer 7 befüllt wird und nur ein Teil der eingebrachten Substanz freigesetzt wird, sodass die Dichtfunktion des Kontaktes zwischen Schürze 7 und nativer Herzklappe 11 durch die in der Kammer 7 verbleibende Substanz gewährleistet ist. Insbesondere ist es vorstellbar, dass eine Substanz aus mehreren Komponenten wie z.B. antimikrobiellen Wirkstoffen, Antibiotika, Antikoagulantien und Kochsalzlösung in die Kammer 7 eingebracht wird, wobei nur ein Teil der Substanz fähig ist, die permeable oder selektiv permeable Schürze per Diffusion in Richtung des Blutes zu übertreten. Die Schürze 6 weist eine spezifische Flexibilität und Elastizität auf. Eine weitere Möglichkeit zur selektiven Freisetzung der eingebrachten Substanz ergibt sich dabei aus einer druckabhängigen Freisetzung, wobei die eingebrachte Substanz 6 freigesetzt wird, bis ein hydrostatischer Grenzdruck in der Kammer 7 oder eine Grenzspannung innerhalb der Schürze 6 erreicht und/oder unterschritten wird. Die Aufrechterhaltung eines minimalen Volumens der Kammer 7 und der paravalvulären Dichtfunktion kann durch die genannten exemplarischen Ausführungsformen sichergestellt werden.

Weiterhin zeigt die Fig. 3, dass eine in die Kammer 7 eingefüllte Substanz nach der Freisetzung aus der Kammer 7 direkt in die Blutbahn und das umliegende Gewebe eintreten kann, um dort beispielsweise als Antibiotikum entzündungshemmend zu wirken. Die vorliegende Erfindung bietet somit den Vorteil, eine interventionelle Behandlung einer Endokarditis direkt an deren Entzündungsquelle im erkrankten Herzen 14 durchführen zu können, ohne den Patienten einer weiteren Belastung neben der Transkatheter-Implantation eines erfindungsgemäßen Systems 2 auszusetzen. Neben der Freisetzung von Medikamenten, wie z.B. verschiedenen Antibiotika, ist gleichfalls auch die Verwendung zur Freisetzung von Antikoagulantien oder weiteren Substanzen zur interventionellen Therapie möglich. Als weitere Freisetzungsmechanismen, abgesehen von der mindestens einen, mit einer Substanzen befüllten Kammer 7, sollen im Weiteren auch Oberflächenbeschichtungen des Systems 2 und biologisch degradierbare Material, z.B. in Form von resorbierenden Matrizen, innerhalb der Kammer 7 für die interventionelle Therapie durch die vorliegende Erfindung in Betracht gezogen werden.

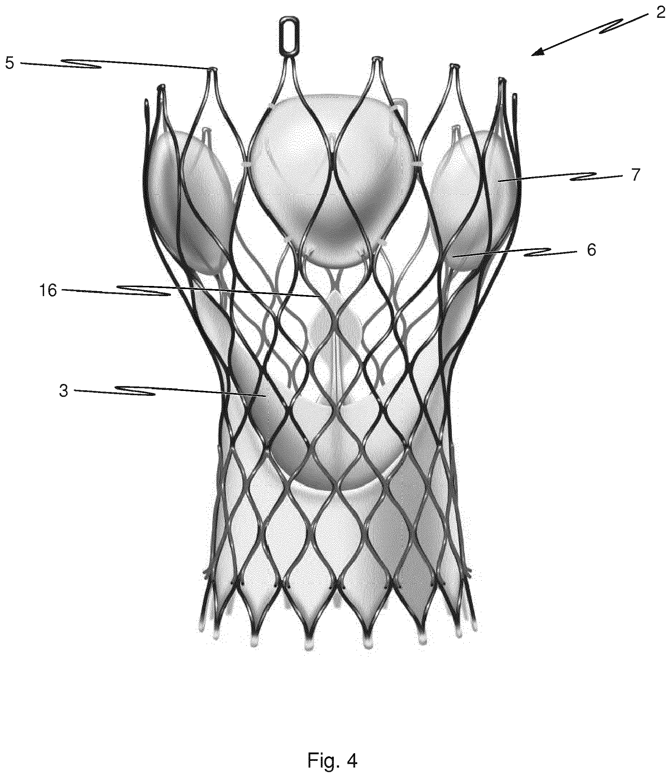

Fig. 4 veranschaulicht eine weitere Ausführungsform des erfindungsgemäßen Systems 2 im expandierten Zustand des mindestens einen Stents. Dabei ist an dem einen Stent 5 eine Ersatzherzklappe 3 befestigt. Des Weiteren ist ein Schürzenbereich 6 im aortenseitigen Retentionsbereich des Stents 5 vorgesehen, wobei in diesem Fall mehrere einzelne Kammern 7 an der Innenseite des Stents 5 vorgesehen sind. Jede der Kammern 7 weist dabei mindestens eine strömungsmäßige Verbindung 16 zum ventrikelseitigen Retentionsbereich des Stents 5 auf, um dort die Freisetzung einer in die Kammern eingefüllten Substanz zu ermöglichen. Aus den Kammern fließt die jeweils eingebrachte Substanz über die strömungsmäßigen Verbindungen 16 aus den Kammern heraus und verteilt sich vorzugsweise in den Verzweigungen der strömungsmäßigen Verbindungen 16. So wird eine verteilte Freisetzung der Substanz über den gesamten Umfang des Stents 5 ermöglicht. Die Kammern 7 sowie die strömungsmäßigen Verbindungen 16 sind dabei an dem Stent 5 mit Hilfe einer Verklammerung, Vernähung oder einer vergleichbaren Befestigungsmöglichkeit fixiert.

### BEZUGSZEICHENLISTE

- 1:: Kathetereinführsystem
- 2:: System
- 3:: Herzklappenersatz
- 4:: Herzklappen-Leaflet
- 5:: Stent
- 6:: Schürze
- 7:: Kammer
- 9:: Katheterspitze
- 10:: Ballon
- 11:: native Herzklappe
- 12:: Katheter
- 13:: Kanal
- 14:: biologisches Gewebe (Herz)
- 15:: Bedienelement (extern)
- 16:: Strömungsmäßige Verbindung

## Patentansprüche

1. System (2) zum Ersatz einer an einer Entzündung und/oder einer Infektion erkrankten Klappe des Herzens (14), wobei das System (2) folgendes aufweist:
- ein Stent-System mit mindestens einem expandierbaren Stent (5); und
- eine Ersatzherzklappe (3), die an dem mindestens einen Stent (5) befestigt ist und mindestens zwei Herzklappen-Leaflets (4) aufweist;
wobei der mindestens eine Stent (5) vorzugsweise an der Innenseite und/oder der Außenseite eine Beschichtung mit einer antimikrobiellen Substanz oder einem antimikrobiell wirkenden Trägermaterial aufweist,
**dadurch gekennzeichnet, dass**
das System (2) einen Schürzenbereich (6) aufweist, wobei ein erster Bereich des Schürzenbereiches (6) an der Innenseite und ein zweiter Bereich des Schürzenbereiches (6) an der Außenseite des mindestens einen Stents (5) vorgesehen ist, wobei in oder an dem Schürzenbereich (6) mindestens eine mit einer Substanz befüllbare Kammer (7) vorgesehen ist, welche derart ausgebildet ist, dass die in der Kammer aufgenommene Substanz des mindestens einen Stents (5) an das umliegende Gewebe freisetzbar ist.

2. System (2) nach Anspruch 1,
wobei in dem Schürzenbereich (6) die mindestens eine Kammer (7) in dem ersten Bereich an der Innenseite und/oder in dem zweiten Bereich an der Außenseite des mindestens einen Stents (5) ausgebildet ist.

3. System (2) nach Anspruch 2,
wobei die Kammer (7) vor und/oder während und/oder nach der Implantation des mindestens einen Stents (5) mit einer Substanz befüllbar ist.

4. System (2) nach einem der Ansprüche 1 bis 3,
wobei die Schürze (6) eine permeable Membran, insbesondere eine selektiv permeable Membran aufweist.

5. System (2) nach Anspruch 2 oder 3,
wobei die mindestens eine Kammer (7) mindestens eine strömungsmäßige Verbindung (16) zum Kontaktbereich zum umliegenden Gewebe des mindestens einen Stents (5) aufweist.

6. System (2) nach einem der Ansprüche 1 bis 5,
wobei die Substanz eine antimikrobiell wirkende Substanz ist und/oder mindestens eine antimikrobiell wirkende Komponente aufweist.

7. System (2) nach einem der Ansprüche 1 bis 6,
wobei ein Kathetereinführsystem (1) vorgesehen ist zum Implantieren des mindestens einen Stents (5).

8. System (2) nach Anspruch 7,
wobei das Kathetereinführsystem (1) ausgebildet ist zum transapikalen oder transfemoralen Einführen des mindestens einen Stents (5) und der daran befestigten Ersatzherzklappe (3).

9. System (2) nach Anspruch 7 oder 8,
wobei das Kathetereinführsystem (1) mindestens einen, vorzugsweise lösbar mit dem Kathetereinführsystem (1) verbundenen Kanal (13) zum Befüllen der mindestens einen Kammer (7) mit einer Substanz aufweist, vorzugsweise im implantierten Zustand des Stents (5) und der daran befestigten Ersatzherzklappe (3).

10. System (2) nach Anspruch 9,
wobei der mindestens eine Kanal (13) lösbar mit der mindestens einen Kammer (7) verbunden ist.

11. System (2) nach einem der Ansprüche 1 bis 10,
wobei die antimikrobielle Beschichtung des mindestens einen Stents (5) nach dem Implantieren vorzugsweise kontrolliert aktivierbar ist.

## Claims

1. A system (2) for replacing an inflamed and/or infected valve of the heart (14), wherein the system (2) comprises the following:
- a stent system comprising at least one expandable stent (5); and
- a replacement heart valve (3) which is affixed to the at least one stent (5) and comprises at least two heart valve leaflets (4);
wherein the at least one stent (5) preferably has an interior and/or exterior coating comprising an antimicrobial substance or an antimicrobial-acting su bstrate,
**characterized in that**
the system (2) comprises a skirt region (6), wherein a first area of the skirt region (6) is provided on the inner side and a second area of the skirt region (6) is provided on the outer side of the at least one stent (5), wherein at least one chamber (7) able to be filled with a substance is provided in or on the skirt region (6) which is designed such that the substance accommodated in the chamber of the at least one stent (5) can be released to the surrounding tissue.

2. The system (2) according to claim 1,
wherein the at least one chamber (7) in the skirt region (6) is formed in the first area on the inner side and/or in the second area on the outer side of the at least one stent (5).

3. The system (2) according to claim 2,
wherein the chamber (7) can be filled with a substance before and/or during and/or after implantation of the at least one stent (5).

4. The system (2) according to any one of claims 1 to 3,
wherein the skirt (6) comprises a permeable membrane, in particular a selectively permeable membrane.

5. The system (2) according to claim 2 or 3,
wherein the at least one chamber (7) has at least one fluid connection (16) to the contact area with tissue surrounding the at least one stent (5).

6. The system (2) according to any one of claims 1 to 5,
wherein the substance is an antimicrobial-acting substance and/or comprises at least one antimicrobial-acting component.

7. The system (2) according to any one of claims 1 to 6,
wherein a catheter delivery system (1) is provided for the implanting of the at least one stent (5).

8. The system (2) according to claim 7,
wherein the catheter delivery system (1) is designed for the transapical or transfemoral insertion of the at least one stent (5) and the replacement heart valve (3) affixed thereto.

9. The system (2) according to claim 7 or 8,
wherein the catheter delivery system (1) comprises at least one channel (13), connected preferably detachably to the catheter delivery system (1), for filling the at least one chamber (7) with a substance, preferably in the implanted state of the stent (5) and the replacement heart valve (3) affixed thereto.

10. The system (2) according to claim 9,
wherein the at least one channel (13) is detachably connected to the at least one chamber (7).

11. The system (2) according to any one of claims 1 to 10,
wherein the antimicrobial coating of the at least one stent (5) can be activated preferably in controlled manner subsequent implanting.

## Revendications

1. Système (2) de remplacement d'une valvule cardiaque (14) atteinte d'une inflammation et/ou d'une infection, le système (2) comprenant :
- un système de stent comportant au moins un stent expansible (5) ; et
- une valvule cardiaque de remplacement (3) qui est fixée sur ledit au moins un stent (5) et qui comprend au moins deux feuillets valvulaires (4) ;
ledit au moins un stent (5) présentant de préférence sur le côté intérieur et/ou sur le côté extérieur un revêtement pourvu d'une substance antimicrobienne ou d'un matériau porteur à effet antimicrobien,
**caractérisé en ce que**
le système (2) comprend une région formant jupe (6), une première zone de la région formant jupe (6) étant prévue sur le côté intérieur et une seconde zone de la région formant jupe (6) étant prévue sur le côté extérieur dudit au moins un stent (5), au moins une chambre (7) remplissable d'une substance étant prévue dans ou sur la région formant jupe (6) et étant réalisée de telle sorte que la substance dudit au moins un stent (5) reçue dans la chambre est susceptible d'être dégagée vers les tissus environnants.

2. Système (2) selon la revendication 1,
dans lequel, dans la région formant jupe (6), ladite au moins une chambre (7) est réalisée dans la première zone sur le côté intérieur et/ou dans la seconde zone sur le côté extérieur dudit au moins un stent (5).

3. Système (2) selon la revendication 2,
dans lequel la chambre (7) est remplissable d'une substance avant et/ou pendant et/ou après l'implantation dudit au moins un stent (5).

4. Système (2) selon l'une des revendications 1 à 3,
dans lequel la jupe (6) comprend une membrane perméable, en particulier une membrane perméable sélectivement.

5. Système (2) selon la revendication 2 ou 3,
dans lequel ladite au moins une chambre (7) comprend au moins une communication fluidique (16) avec la zone de contact vers les tissus environnants dudit au moins un stent (5).

6. Système (2) selon l'une des revendications 1 à 5,
dans lequel la substance est une substance à effet antimicrobien et/ou comprend au moins une composante à effet antimicrobien.

7. Système (2) selon l'une des revendications 1 à 6,
dans lequel il est prévu un système d'introduction de cathéter (1) pour implanter ledit au moins un stent (5).

8. Système (2) selon la revendication 7,
dans lequel le système d'introduction de cathéter (1) est réalisé pour l'introduction transapicale ou transfemorale dudit au moins un stent (5) et de la valvule cardiaque de remplacement (3) fixée sur celui-ci.

9. Système (2) selon la revendication 7 ou 8,
dans lequel le système d'introduction de cathéter (1) comprend au moins un canal (13) relié de préférence de façon détachable au système d'introduction de cathéter (1) et destiné à remplir ladite au moins une chambre (7) d'une substance, de préférence dans l'état implanté du stent (5) et de la valvule cardiaque de remplacement (3) fixée sur celui-ci.

10. Système (2) selon la revendication 9,
dans lequel ledit au moins un canal (13) est relié de façon détachable à ladite au moins une chambre (7).

11. Système (2) selon l'une des revendications 1 à 10,
dans lequel après implantation, le revêtement antimicrobien dudit au moins un stent (5) est activable de préférence de façon contrôlée.
